(19) Europäisches Patentamt
European Patent Office
Office européen des brevets

(11) Publication number: **0 264 978 B1**

(12) **EUROPEAN PATENT SPECIFICATION**

(45) Date of publication of patent specification: **07.08.91**

(51) Int. Cl.5: **C07D 493/04**, C07D 307/20, C07D 407/12, C07D 405/12, C07D 409/12, A01N 43/90, //(C07D493/04,307:00,307:00)

(21) Application number: **87201574.8**

(22) Date of filing: **19.08.87**

(54) **Heterocyclic herbicides.**

(30) Priority: **22.08.86 US 899285**

(43) Date of publication of application:
**27.04.88 Bulletin 88/17**

(45) Publication of the grant of the patent:
**07.08.91 Bulletin 91/32**

(84) Designated Contracting States:
**AT BE CH DE ES FR GB GR IT LI LU NL SE**

(56) References cited:
**DE-A- 2 709 144**
**US-A- 4 169 152**
**US-A- 4 400 198**
**US-A- 4 429 119**
**US-A- 4 515 618**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Sun, King Mo**
**512 Bellwood Lane**
**Modesto California 95356(US)**

(74) Representative: **Bennett, David Arthur Horder et al**
**4, York Road**
**London SE1 7NA(GB)**

**EP 0 264 978 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to certain 2,6-dioxabicyclo-(3.3.0)octane ether derivatives, the preparation thereof, their use as herbicides and plant growth regulators and to compositions containing these ether derivatives for such uses.

Certain furanose derivatives are described in U.S. patents 4,429,119, 4,497,649, 4,515,618, 4,534,785, 4,554,010 and 4,579,582 as herbicides and plant growth regulators. Japanese patents 55/154,992 and 55/164,992 also disclose certain furanose derivatives as intermediates to antibiotic Cerulenin used to study fatty acid synthesis in plants.

There has now been discovered a novel class of dioxabicyclooctane ethers, which may be prepared from such furanose derivatives, and which exhibit useful herbicidal and plant growth regulatory activity, especially in respect of grassy weeds.

.The present invention is directed to novel compounds of the formula I

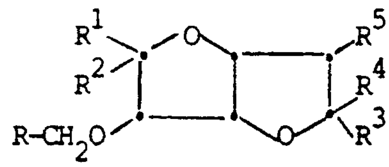

(I)

wherein $R^1$, $R^2$, $R^3$, $R^4$, and $R^5$, which may be the same or different, each independently is a hydrogen atom, or an alkyl group containing 1 to 5 carbon atoms optionally substituted by one or more halogen atoms having an atomic number of from 9 to 35; or $R^3$, $R^4$ and $R^5$ are additionally selected from an alkenyl or alkynyl group containing 2 to 4 carbon atoms and a mono- or bicyclic cycloaliphatic or aryl group containing 6 to 10 carbon atoms; or $R^3$ and $R^4$ together with the carbon atom to which they are attached form a cycloaliphatic ring containing 5 or 6 ring carbon atoms; and R is selected from an unsaturated hydrocarbon group containing 2 to 4 carbon atoms, an aromatic group containing up to 14 carbon atoms, a heterocyclic group having one or two heteroatoms each selected from O, S or N and containing up to 14 carbon atoms or an N-oxide or acid addition salt of an N-heterocyclic group, a cyano group, a cycloalkenyl group containing 5 to 7 carbon atoms and a cycloalkyl group containing 3 to 10 carbon atoms, which groups are optionally substituted by one or more substituents selected from the groups hydroxy; cyano; halogen atoms having an atomic number of from 9 to 35 inclusive;
alkyl, optionally substituted by hydroxy, amino, alkylcarbonylamino, alkoxy or alkylthio; haloalkyl, alkoxy, haloalkoxy, alkylthio, haloalkylthio, alkylsulfinyl, alkylsulfonyl, alkenyl or alkynyl of up to 4 carbon atoms; carboxyl; aminocarbonyl or amino or each of which can be substituted on the nitrogen by one or two alkyl groups of 1 to 4 carbon atoms; and stereoisomeric forms or mixtures thereof.

The compounds of formula I have been found useful for influencing plant growth and controlling the growth of unwanted plants, being particularly active with respect to grassy weeds. For example, the compounds can change plant morphology; depress the growth of plants, such as broadleafed weeds; inhibit germination; or totally or selectively kill plants depending on the amount used. As herbicides, they appear to be more effective when applied pre-emergence or pre-plant incorporated (applied to the soil before the seeds have sprouted) than when applied post-emergence (applied to the foliage).

The preferred optional substituent for R is an alkyl group containing 1 or 2 carbon atoms, especially a methyl group.

In one preferred group of the compounds of formula I, R is selected from an ethynyl group, a phenyl group optionally substituted by one or two halogen atoms of atomic number 9 or 17 or by a methyl group, and a 5- or 6-membered heterocyclic group containing one or two heteroatoms of 0, S or N, optionally ring-substituted on a carbon atom by halogen of atomic number 9 or 17 or by alkyl containing 1 or 2 carbon atoms. Preferably, R is phenyl, 2,6-difluorophenyl, 2,6-dichlorophenyl, 2-methylphenyl, 2-fluorophenyl, tetrahydro-2-pyranyl, 6-chloro-2-pyridinyl or 3-chloro-2-thienyl.

In another embodiment of the compounds of formula I, $R^1$, $R^2$, $R^3$, $R^4$ and $R^5$ each independently is a hydrogen atom or an alkyl group containing 1 or 2 carbon atoms optionally substituted by one or more halogen atoms, e.g. methyl, ethyl and chloromethyl. Preferably, $R^1$ is methyl or ethyl and preferably $R^2$, $R^3$, $R^4$ and $R^5$ are hydrogen.

As stated above, the invention includes N-oxides and salts of the compounds of formula I in which R is an N-containing heterocyclic group. A salt is formed from any non-phytotoxic anion for example, an acid addition salt or a quarternary ammonium salt, for example a compound of the general formula:

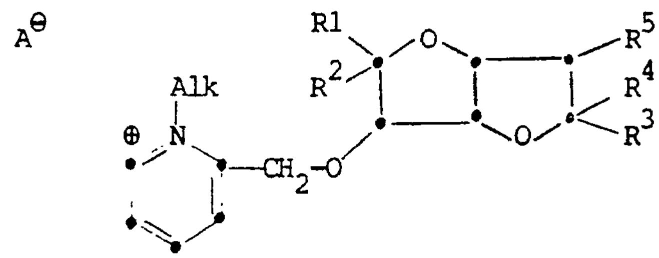

in which Alk represents an alkyl group having up to 6 carbon atoms, especially a methyl or an ethyl group, and $A^{\ominus}$ represents one equivalent of an anion, especially a halogen ion, for example an iodide or chloride ion, or one equivalent of a sulphate ion. From the halides, other non-phytotoxic salts can be prepared by known methods, such as the nitrate, sulfate, trifluoromethansulfonate, chlorate, acetate, borate, tartrate, succinate and phosphate.

The compounds of formula I exhibit geometrical and optical isomerism and may be prepared in geometrical and/or optically active forms, and as racemates. The various individual optical and geometrical forms and various combinations thereof of the materials of the invention usually have some difference in herbicidal properties. The present invention contemplates all the herbicidally active forms resulting from synthesis, as well as deliberately created mixtures.

For example, the compounds of the invention of the formula

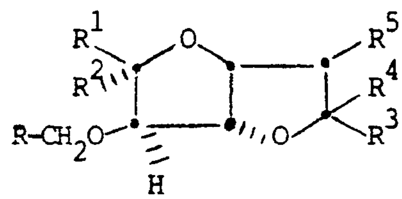

are herbicidal. They can be prepared from the corresponding compounds wherein $R^1$ and $OCH_2$-R are in trans configuration with respect to the plane of the furan ring by a three step process of hydrolysis to remove the $CH_2R$ group, epimerization of the remaining hydroxyl group by standard methods, to the opposite stereoconfiguration and reformation of the ether group $OCH_2R$ by treatment with the appropriate halide $RCH_2X$ in which X is a halogen atom.

The compounds of formula I may be prepared from the corresponding furanose derivatives of the formula II

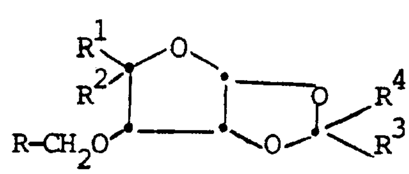

(II)

wherein R, $R^1$, $R^2$, $R^3$ and $R^4$ have the meanings defined in formula I or its corresponding glycol derivative of formula III

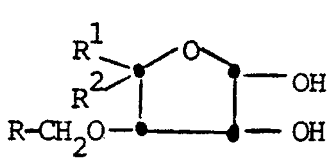

(III)

which can be obtained from the furanose derivative of Formula II by hydrolysis, e.g. with a strong acid such

as sulfuric acid. These furanose derivatives are known in the art as in the above-mentioned U.S. patents 4,429,119, 4,497,649 4,515,618, 4,554,010 and 4,579,582 and Japanese patents 55/154,992 and 55/154,993.

The glycol derivatives of formula III are condensed, suitably by heating in an inert solvent, e.g. acetonitrile, with a suitable agent, such as carbomethoxymethylenetriphenylphosphorane optionally substituted on the methylene carbon, to form a lactone of formula IV

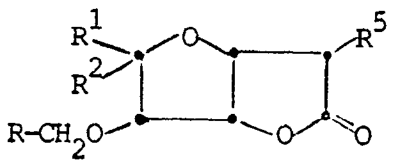

(IV)

The above lactone may be reduced in the presence of a conventional hydride or metal reducing agent, e.g. lithium aluminium hydride, to reduce the keto group to a hydroxy group and provide a diol compound of formula V.

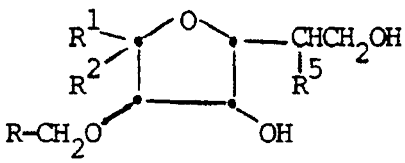

(V)

The reaction is conveniently conducted under anhydrous conditions at ambient temperature in the presence of an inert solvent, such as diethyl ether.

The above diol V is subjected to a ring closure in the presence of, e.g. benzenesulfonyl chloride at low temperatures, preferably in the presence of an inert solvent, such as pyridine, to provide compounds of formula I wherein $R^3$ and $R^4$ are hydrogen.

For compounds of formula I wherein $R^3$ and/or $R^4$ is other than H, the lactone IV is treated with at least one alkyl metal, suitably of formula $R^3M$, $R^4M$ or $R^3R^4M$ where M represents a metal (e.g. $R^2Li$ and/or $R^4Li$), suitably at low temperature (<-30°C), preferably in an inert solvent (e.g. diethyl ether), or lactone IV is first treated with an alkyl metal (e.g. $R^3Li$) and then with a reducing agent such as lithium aluminium hydride in an inert solvent at low temperature, to provide a diol of formula VI

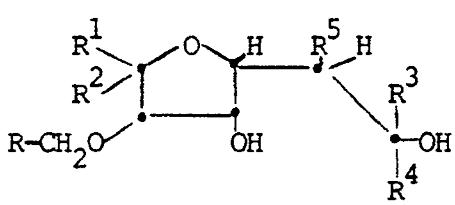

(VI)

For $R^3$ and/or $R^4$ other than hydrogen ring closure may be effected by subjecting diol VI to a pre-mix of dimethyl sulfoxide and trimethylsilyl chloride in an inert solvent, e.g. toluene, at 40°-45°C for about 24 hours, to give corresponding compounds of formula I.

For application, a compound of formula I ordinarily is applied most effectively by formulating it with a suitable inert carrier or surface-active agent, or both. The invention, therefore, also includes compositions suitable for combatting unwanted plants, such compositions comprising an inert carrier or surface-active agent, or both, and, as active ingredient, at least one compound of formula I.

The term "carrier" as used herein means an inert solid or liquid material, which may be inorganic or organic and of synthetic or natural origin, with which the active compound is mixed or formulated to faciliate its application to the plant, seed, soil or other object to be treated, or its storage, transport and/or handling. Any of the materials customarily employed in formulating pesticides, herbicicdes, or fungicides -- i.e., horticulturally acceptable carriers -- are suitable.

Suitable solid carriers are natural and synthetic clays and silicates, for example, natural silicas such as diatomaceous earths; magnesium silicates, for example talcs; magnesium aluminium silicates, for example, attapulgites and vermiculites; aluminium silicates, for example, kaolinites, montmorillonites and micas; calcium carbonate; calcium sulfate; synthetic hydrated silicon oxides and synthetic calcium or aluminium silicates; elements such as; for example, carbon and sulfur; natural and synthetic resins such as, for example coumarone resins, polyvinyl chloride and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Examples of suitable liquid carriers are water, alcohols such as isopropyl alcohol and glycols; ketones such as acetone, methyl ethyl ketone, methyl isobutyl ketone and cyclohexanone; ethers such as cellosolves; aromatic hydrocarbons such as benzene, toluene and xylene; petroleum fractions such as kerosene and light mineral oils; chlorinated hydrocarbons such as carbon tetrachloride, perchloroethylene and trichloromethane. Also suitable are liquefied, normally vaporous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions of the invention may be prepared as wettable powders, dusts, granules, solutions, emulsifiable concentrates, emulsions, suspension concentrates and aerosols. Wettable powders are usually compounded to contain 25 to 75% by weight of active compound and usually contain, in addition to the solid carrier, 3-10% by weight of a dispersing agent, 2-15% of a surface-active agent and, where necessary, 0-10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0.5-10% by weight of the active compound. Granules are usually prepared to have a size between 10 and 100 BS mesh (1.676-0.152mm), and may be manufactured by agglomeration or impregnation techniques. Generally, granules will contain 0.5-25% by weight of the active compound, and 0-1% by weight of additives such as stabilizers, slow release modifiers and binding agents. Emulsifiable concentrates usually contain, in addition to the solvent and, when necessary, cosolvent, 10-50% weight per volume of the active compound, 2-20% weight per volume emulsifiers and 0-20% weight per volume of appropriate additives such as stabilizers, penetrants and corrosion inhibitors. Suspension concentrates are compounded so as to obtain a stable, non-sedimenting, flowable product and usually contain 10-75% weight of the active compound, 0.5-5% weight of dispersing agents, 1-5% of surface-active agent, 0.1-10% weight of suspending agents, such as defoamers, corrosion inhibitors, stabilizers, penetrants and stickers, and as carrier, water or an organic liquid in which the active compound is substantially insoluble; certain organic solids or inorganic salts may be dissolved in the carrier to assist in preventing sedimentation or as antifreeze agents for water.

Of particular interest in current practice are water-dispersable granular formulations. These are in the form of dry, hard granules that are essentially dust-free, and are resistant to attrition on handling, thus minimizing the formation of dust. On contact with water, the granules readily disintegrate to form stable suspensions of the particles of active material. Such formulations contain 90% or (up to 95%) more by weight of finely divided active material, 3-7% by weight of a blend of surfactants, which act as wetting, dispersing, suspending and binding agents, and may contain up to 3% by weight of a finely divided carrier, which acts as a resuspending agent.

Aqueous dispersions and emulsions, for example, compositions obtained by diluting a wettable powder or a concentrate according to the invention with water, also lie within the scope of the present invention. The said emulsions may be of the water-in-oil or of the oil-in-water type, and may have thick, mayonnaise-like consistency.

It is evident from the foregoing that this invention contemplates compositions containing as little as about 0.5% by weight to as much as about 95% by weight of a compound of formula I as the active ingredient.

The compositions of the invention may also contain other ingredients, for example, other compounds posessing pesticidal, especially insecticidal, acaricidal, herbicidal or fungicidal properties, as are appropriate to the intended purposes.

Protection of a locus or area from undesirable plants, especially grassy weeds, is effected by applying a compound of formula I, ordinarily in a composition of one of the aforementioned types, to soil in which the seeds of the unwanted plants are present, or to the foilage of the unwanted plants. The active compound, of course, is applied in an amount sufficient to exert the desired action.

The amount of compound of the invention to be used in combatting undesired plants will naturally depend on the condition of the plants, the degree of activity desired, the formulation used, the mode of application, the climate, the season of the year, and other variables. Recommendations as to precise amounts are, therefore, not possible. In general, however, application to the locus to be protected of from 0.1 to 10.0 kg per hectare of the compound of formula I will be satisfactory.

## Examples

The invention is illustrated by the following examples. The products, including intermediates, were identified by elemental, infrared, or nuclear magnetic resonance (NMR) spectral analyses as necessary.

### Example 1 3-0-Benzyl-5,6-dideoxy-D-xylo-hexofuranose

3-0-Benzyl--5,6-dideoxy-1,2-0-isopropylidene-alpha-D-xylo-hexofuranoside (50 g) was dissolved in dioxane (400 ml) and dilute sulfuric acid (10% by weight, 50ml) and water (200 ml) were added. The mixture was heated in a steam bath for 4 hours, cooled to room temperature, and added slowly to an aqueous sodium bicarbonate solution. Enough sodium bicarbonate was used to bring the pH of the mixture to neutrality. The mixture was extracted with methylene chloride thoroughly. The combined organic extracts were dried over anhydrous magnesium sulfate and concentrated under reduced pressure to give 3-0-benzyl-5,6-dideoxy-D-xylo-hexofuranose as a thick syrup (43 g) $[\alpha]_D^{20}$ = -10.43($CHCl_3$).

### Example 2 5-0-Benzyl-1,4;3,6-didehydro-2,7,8-trideoxy-D-ido-octonate

3-0-Benzyl-5,6-dideoxy-D-xylo-hexofuranose (6.6g) and carbomethoxymethylenetriphenylphosphorane (16g) were refluxed in acetonitrile (200 ml) for 14 hours. The solvent was removed. The residue was placed at the top of a silica gel column and eluted with a mixture of ethyl acetate (50%) and hexane (50%) to give the desired product (3.6g) $[\alpha]_D^{20}$ = -14.5($CHCl_3$).

### Example 3 5-0-Benzyl-3,6-dehydro-2,7,8-trideoxy-D-ido-octitol

The octonate of Example 2 above (2 g) was dissolved in anhydrous diethyl ether (30 ml) and lithium aluminum hydride (0.14 g) was added in portions. The mixture was stirred at room temperature for 30 minutes. Excess hydrated sodium sulfate was added and the mixture was stirred vigorously for 30 minutes. It was filtered and the filtrate was concentrated to an oil which was purified on a silica gel column eluted with ethyl acetate to give the desired product (1.9 g). $[\alpha]_D^{20}$ = -37.9 ($CHCl_3$).

### Example 4 5-0-Benzyl-1,4;3,6-didehydro-2,7,8-trideoxy-D-ido-octitol

To the octitol of Example 3 above (0.2 g) in pyridine (3 ml) at -5°C, was added benzenesulfonyl chloride (0.135 g). The mixture was stirred at -5°C for 14 hours. It was then poured into dilute hydrochloric acid, and extracted with diethyl ether. The combined diethyl ether extracts were washed with sodium bicarbonate solution and water. The organic layer was dried over anhydrous magnesium sulfate and concentrated to a syrup which was purified on a silica gel column eluted with diethyl ether to give the desired product (0.11 g).

### Example 5-16

Following procedures similar to those described in Examples 1-4, the following representative species

are prepared as set forth in Table 1.

Table 1.

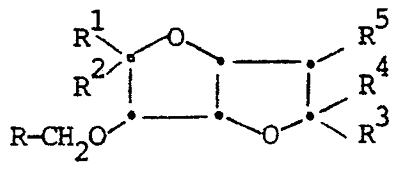

(I)

| Example | R | $R^1$ | $R^2$ | $R^3$ | $R^4$ | $R^5$ |
|---|---|---|---|---|---|---|
| 5 | 2-$CH_3$phenyl | H | H | H | H | H |
| 6 | 2,6-$F_2$phenyl | H | H | H | H | H |
| 7 | 2,6-$Cl_2$phenyl | H | H | H | H | H |
| 8 | 3-Cl-2-thienyl | H | H | H | H | H |
| 9 | tetrahydro-2-pyranyl | H | H | H | H | H |
| 10 | 2-pyridinyl | H | H | H | H | H |
| 11 | phenyl | $CH_3$ | H | H | H | H |
| 12 | 2-$CH_3$phenyl | $C_2H_5$ | H | $C_2H_5$ | $C_2H_5$ | H |
| 13 | 2-Fphenyl | $CH_3$ | $CH_3$ | $CH_3$ | $CH_3$ | $CH_3$ |
| 14 | tetrahydro-2-pyranyl | $CH_2Cl$ | H | H | H | H |
| 15 | 2-Fphenyl | H | H | H | H | H |
| 16 | phenyl | H | H | $-(CH_2)_5-$ | | H |

Example 17 Herbicidal activity

The preemergence (soil) herbicidal activity of compounds of formula I w as evaluated by planting seeds of downy brome, johnson-grass, yellow foxtail, barnyardgrass, yellow millet, blackgrass, hemp sesbania, velvetleaf, morningglory, prickly sida, sicklepod and garden cress in test tubes, nominally measuring 25 × 200 millimeters, filled about three-quarters full of untreated soil, in each case covered on top with about 2.5 cubic centimeters of soil treated with 0.1 milligram of the test compound, to give a dosage of 2.24 kg of the test compound per hectare (2.0 pounds per acre). The seeds were planted on top of the treated soil and covered with about 1.5 cubic centimeters of untreated soil. The planted soil was held under a controlled regimen of temperature, moisture, and light. At 10 days, the amount of germination and growth in each tube were evaluated on a 0 to 9 scale, the numerical ratings having the following meanings:

| Rating | Meaning |
|---|---|
| 9 | No living tissue |
| 8 | Plant severely damaged and expected to die |
| 7 | Plant badly damaged, but expected to live |
| 6 | Moderate damage, but complete recovery expected |
| 5 | Intermediate damage (probably unacceptable for crop plants) |
| 3-4 | Observable damage |
| 1-2 | Plant slightly affected, possibly by the chemical, possibly due to biological variability |
| 0 | No visible effect |

In the above pre-emergence test, a representative compound of the invention, Compound 4, had ratings of 8 or 9 against downy brome, johnsongrass, yellow foxtail, barnyardgrass, yellow millet and blackgrass and showed no visible activity on the other plant species tested.

## Claims

### Claims for the following Contracting States : BE, CH, DE, FR, GB, GR, IT LI, LU, NL, SE

1. A compound of the formula I

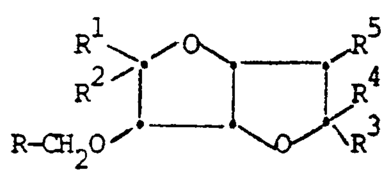

(I)

wherein $R^1$, $R^2$, $R^3$, $R^4$ and $R^5$, which may be the same or different, each independently is a hydrogen atom, or an alkyl group containing 1 to 5 carbon atoms optionally substituted by one or more halogen atoms having an atomic number of from 9 to 35; or $R^3$, $R^4$ and $R^5$ are additionally selected from an alkenyl or alkynyl group containing 2 to 4 carbon atoms and a mono- or bicyclic cycloaliphatic or aryl group containing 6 to 10 carbon atoms; or $R^3$ and $R^4$ together with the carbon atom to which they are attached form a cycloaliphatic ring containing 5 or 6 ring carbon atoms; and R is selected from an unsaturated hydrocarbon group containing 2 to 4 carbon atoms, an aromatic group containing up to 14 carbon atoms, a heterocyclic group having one or two heteroatoms each selected from O, S or N and containing up to 14 carbon atoms or an N-oxide or acid addition salt of an N-heterocyclic group, a cyano group, a cycloalkenyl group containing 5 to 7 carbon atoms and a cycloalkyl group containing 3 to 10 carbon atoms, which groups are optionally substituted by one or more substituents selected from the groups hydroxy; cyano; halogen atoms having an atomic number of from 9 to 35 inclusive; alkyl, optionally substituted by hydroxy, amino, alkylcarbonylamino, alkoxy or alkylthio; haloalkyl, alkoxy, haloalkoxy, alkylthio, haloalkylthio, alkylsulfinyl, alkylsulfonyl, alkenyl or alkynyl of up to 4 carbon atoms; carboxyl; aminocarbonyl or amino, each of which can be substituted on the nitrogen by one or two alkyl groups of 1 to 4 carbon atoms; and stereoisomeric forms or mixtures thereof.

2. A compound according to claim 1 wherein R is selected from an ethynyl group; a phenyl group optionally substituted by one or two halogen atoms of atomic number 9 or 17 or by a methyl group, and a 5- or 6- membered heterocyclic group containing one to two heterocyclic atoms of O, S or N, optionally ring substituted on a carbon atom by halogen of atomic number 9 or 17 or by alkyl containing 1 or 2 carbon atoms.

3. A compound accerding to claim 2 wherein R is phenyl, 2,6-difluorophenyl, 2,6-dichlorophenyl, 2-methylphenyl, 2-fluorophenyl, tetrahydro-2-pyranyl, 6-chloro-2-pyridinyl or 3-chloro-2-thienyl.

4. A compound according to claim 1, 2 or 3 wherein $R^1$, $R^2$, $R^3$, $R^4$ and $R^5$, which may be the same or different, each independently is a hydrogen atom or an alkyl group containing 1 or 2 carbon atoms optionally substituted by one or more halogen atoms.

5. A compound according to any one of claims 1 to 4 wherein $R^1$ is methyl or ethyl.

6. A compound according to any one of claims 1 to 5 wherein $R^2$, $R^3$, $R^4$ and $R^5$ are each hydrogen.

7. A compound according to claim 1 wherein R is phenyl, $R^1$ is ethyl and $R^2$ , $R^3$ , $R^4$ and $R^5$ are each hydrogen.

8. A compound of the formula IV

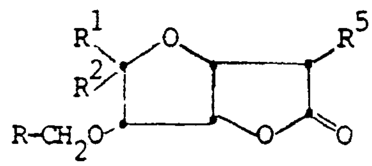

(IV)

where R, $R^1$, $R^2$ and $R^5$ are as defined in any one of claims 1 to 7; and stereoisomeric forms or mixtures thereof.

9. A compound of the formula VI

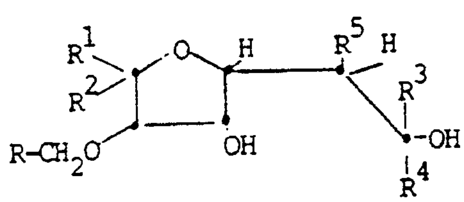

(VI)

where R, $R^1$, $R^2$, $R^3$, $R^4$ and $R^5$ are as defined in any one of claims 1 to 7 and stereoisomeric forms and mixtures thereof.

10. A process for the preparation of a compound of formula I where $R^3$ and $R^4$ are each hydrogen comprising reducing a lactone of formula IV as defined in claim 8 to provide a diol of formula V

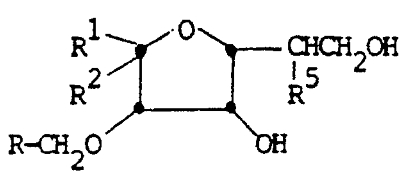

(V)

where R, $R^1$, $R^2$ and $R^5$ are as defined in claim 1, and subjecting the diol of formula V to conditions effecting ring closure to give a compound of formula I where $R^3$ and $R^4$ are each hydrogen.

11. A process for the preparation of a compound of formula I where $R^3$ and/or $R^4$ is other than hydrogen comprising reacting a lactone of formula IV as defined in claim 8 with at least one alkyl metal of formula $R^3M$, $R^4M$, or $R^3R^4M$ where $R^3$ and $R^4$ are as defined in claim 1 other than hydrogen and M represents a metal, optionally followed by treatment with a reducing agent, to give a compound of

formula VI as defined in claim 9 where $R^3$ and/or $R^4$ is other than hydrogen and subjecting the diol of formula VI to conditions effecting ring closure to give a compound of formula I where $R^3$ and/or $R^4$ is other than hydrogen.

**12.** A process according to claim 10 or 11 wherein the lactone of formula IV has been prepared by condensation of a glycol derivative of formula III

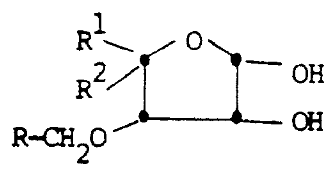

(III)

where R, $R^1$ and $R^2$ are as defined in claim 1, with a suitable condensing agent.

**13.** A process according to claim 12 wherein the glycol derivative of formula III has been obtained by acid hydrolysis of a corresponding furanose derivative.

**14.** A compound of formula I when prepared by the process of any one of claims 10 to 13.

**15.** A composition for plant growth regulation, which comprises a plant growth regulating amount of a compound according to any one of claims 1 to 7 or 14 and at least one surface-active agent or carrier.

**16.** A composition for plant growth regulation, which comprises a compound according to any one of claims 1 to 7 or 14 and at least one surface-active agent and/or carrier.

**17.** A method for regulating plant growth at a locus which comprises applying to the locus a compound according to any one of claims 1 to 7 or 14 or a composition according to claim 15 or 16.

**18.** A method according to claim 17 wherein the regulation results in killing of the plant.

**Claims for the following Contracting State : AT**

**1.** A composition for plant growth regulation comprising at least one surface-active agent or carrier and a compound of the formula I

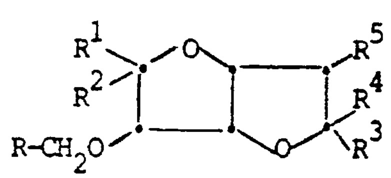

(I)

wherein $R^1$, $R^2$, $R^3$, $R^4$ and $R^5$, which may be the same or different, each independently is a hydrogen atom, or an alkyl group containing 1 to 5 carbon atoms optionally substituted by one or more halogen atoms having an atomic number of from 9 to 35; or $R^3$, $R^4$ and $R^5$ are additionally selected from an alkenyl or alkynyl group containing 2 to 4 carbon atoms and a mono- or bicyclic cycloaliphatic or aryl group containing 6 to 10 carbon atoms; or $R^3$ and $R^4$ together with the carbon atom to which they are attached form a cycloaliphatic ring containing 5 or 6 ring carbon atoms; and R is selected from an unsaturated hydrocarbon group containing 2 to 4 carbon atoms, an aromatic group containing up to 14 carbon atoms, a heterocyclic group having one or two heteroatoms each selected from O, S or N and containing up to 14 carbon atoms or an N-oxide or acid addition salt of an N-heterocyclic group, a cyano group, a cycloalkenyl group containing 5 to 7 carbon atoms and a cycloalkyl group containing 3 to 10 carbon atoms, which groups are optionally substituted by one or more substituents selected from the groups hydroxy; cyano; halogen atoms having an atomic number of from 9 to 35 inclusive; alkyl, optionally substituted by hydroxy, amino, alkylcarbonylamino, alkoxy or alkylthio; haloalkyl, alkoxy, haloalkoxy, alkylthio, haloalkylthio, alkylsulfinyl, alkylsulfonyl, alkenyl or alkynyl of up to 4 carbon atoms;

carboxyl; aminocarbonyl or amino, each of which can be substituted on the nitrogen by one or two alkyl groups of 1 to 4 carbon atoms; or stereoisomeric forms or mixtures thereof.

**2.** A composition according to claim 1 wherein, in the formula I, R is selected from an ethynyl group; a phenyl group optionally substituted by one or two halogen atoms of atomic number 9 or 17 or by a methyl group, and a 5- or 6- membered heterocyclic group containing one to two heterocyclic atoms of O, S or N, optionally ring substituted on a carbon atom by halogen of atomic number 9 or 17 or by alkyl containing 1 or 2 carbon atoms.

**3.** A composition according to claim 2 wherein, in the formula I, R is phenyl, 2,6-difluorophenyl, 2,6-dichlorophenyl, 2-methylphenyl, 2-fluorophenyl, tetrahydro-2-pyranyl, 6-chloro-2-pyridinyl or 3-chloro-2-thienyl.

**4.** A composition according to claim 1, 2 or 3 wherein, in the formula I, $R^1$, $R^2$, $R^3$, $R^4$ and $R^5$, which may be the same or different, each independently is a hydrogen atom or an alkyl group containing 1 or 2 carbon atoms optionally substituted by one or more halogen atoms.

**5.** A composition according to any one of claims 1 to 4 wherein, in the formula I, $R^1$ is methyl or ethyl.

**6.** A composition according to any one of claims 1 to 5 wherein, in the formula I, $R^2$, $R^3$, $R^4$ and $R^5$ are each hydrogen.

**7.** A composition according to claim 1 wherein, in the formula I, R is phenyl, $R^1$ is ethyl and $R^2$, $R^3$, $R^4$ and $R^5$ are each hydrogen.

**8.** A composition for plant growth regulation which comprises a plant growth regulating amount of a compound of formula I as defined in claim 1 and at least one surface-active agent or carrier.

**9.** A process for the preparation of a compound of formula I as defined in claim 1 where $R^3$ and $R^4$ are each hydrogen, which comprises reducing a lactone of formula IV

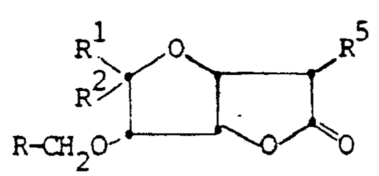

(IV)

to provide a diol of formula V

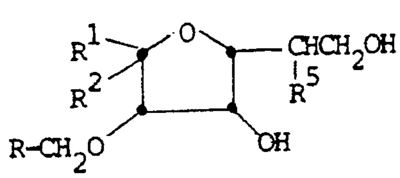

(V)

where R, $R^1$, $R^2$ and $R^5$ are as defined in claim 1, and subjecting the diol of formula V to conditions effecting ring closure to give a compound of formula I where $R^3$ and $R^4$ are each hydrogen.

**10.** A process for the preparation of a compound of formula I as defined in claim 1 where $R^3$ and/or $R^4$ is other than hydrogen which comprises reacting a lactone of formula IV as defined in claim 9 with at least one alkyl metal of formula $R^3M$, $R^4M$ or $R^3R^4M$ where $R^3$ and $R^4$ are as defined in claim 1 other than hydrogen and M represents a metal, optionally followed by treatment with a reducing agent, to give a compound of formula VI

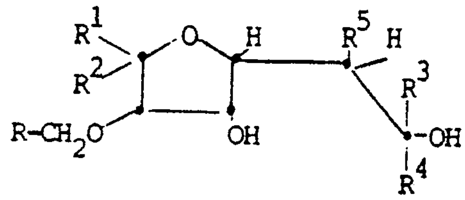

(VI)

where R, $R^1$, $R^2$ and $R^5$ are as defined in claim 1 and where $R^3$ and/or $R^4$ is other than hydrogen and subjecting the diol of formula VI to conditions effecting ring closure to give a compound of formula I where $R^3$ and/or $R^4$ is other than hydrogen.

**11.** A process according to claim 9 or 10 wherein the lactone of formula IV has been prepared by condensation of a glycol derivative of formula III

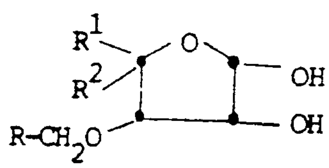

(III)

where R, $R^1$ and $R^2$ are as defined in claim 1, with a suitable condensing agent.

**12.** A process according to claim 11 wherein the glycol derivative of formula III has been obtained by acid hydrolysis of a corresponding furanose derivative.

**13.** A method for regulating plant growth at a locus which comprises applying to the locus a composition according to any one of claims 1 to 8.

**14.** A method according to claim 13 wherein the regulation results in killing of the plant.

**Claims for the following Contracting State : ES**

**1.** A process for the preparation of a compound of the formula I

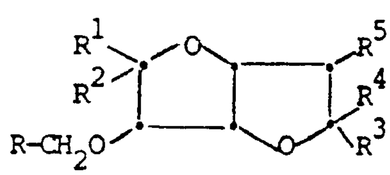

(I)

wherein $R^1$, $R^2$, $R^3$, $R^4$ and $R^5$, which may be the same or different, each independently is a hydrogen atom, or an alkyl group containing 1 to 5 carbon atoms optionally substituted by one or more halogen atoms having an atomic number of from 9 to 35 with the proviso that $R^3$ and/or $R^4$ is other than hydrogen; or $R^3$, $R^4$ and $R^5$ are additionally selected from an alkenyl or alkynyl group containing 2 to 4 carbon atoms and a mono-or bicyclic cycloaliphatic or aryl group containing 6 to 10 carbon atoms; or $R^3$ and $R^4$ together with the carbon atom to which they are attached form a cycloaliphatic ring containing 5 or 6 ring carbon atoms; and R is selected from an unsaturated hydrocarbon group containing 2 to 4 carbon atoms, an aromatic group containing up to 14 carbon atoms, a heterocyclic group having one or two heteroatoms each selected from O, S or N and containing up to 14 carbon atoms or an N-oxide or acid addition salt of an N-heterocyclic group, a cyano group, a cycloalkenyl group containing 5 to 7 carbon atoms and a cycloalkyl group containing 3 to 10 carbon atoms, which groups are optionally substituted by one or more substituents selected from the groups hydroxy; cyano; halogen atoms having an atomic number of from 9 to 35 inclusive; alkyl, optionally substituted by hydroxy, amino, alkylcarbonylamino, alkoxy or alkylthio; haloalkyl, alkoxy, haloalkoxy, alkylthio, haloalkylthio, alkylsulfinyl, alkylsulfonyl, alkenyl or alkynyl of up to 4 carbon atoms; carboxyl; aminocarbonyl or amino, each of which can be substituted on the nitrogen by one or two alkyl groups of 1 to 4 carbon atoms; and stereoisomeric forms or mixtures thereof, which comprises reacting a lactone of formula IV

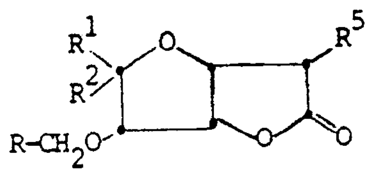

(IV)

with at least one alkyl metal of formula $R^3M$, $R^4M$ or $R^3R^4M$, where R, $R^1$, $R^2$, $R^3$, $R^4$ and $R^5$ are as hereinbefore defined and M represents a metal, optionally followed by treatment with a reducing agent, to give a compound of formula VI

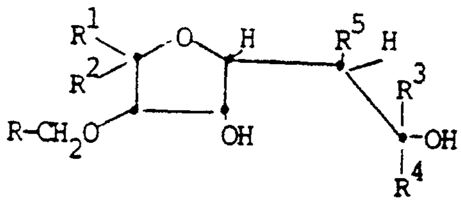

(VI)

and subjecting the diol of formula VI to conditions effecting ring closure to give a compound of formula I.

2. A process for the preparation of a compound of the formula I

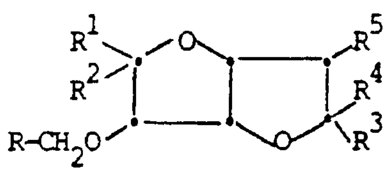

(I)

wherein $R^3$ and $R^4$ each represents a hydrogen atom; $R^1$, $R^2$ and $R^5$, which may be the same or different, each independently is a hydrogen atom, or an alkyl group containing 1 to 5 carbon atoms optionally substituted by one or more halogen atoms having an atomic number of from 9 to 35; or $R^5$ is additionally selected from an alkenyl or alkynyl group containing 2 to 4 carbon atoms and a mono- or bicyclic cycloaliphatic or aryl group containing 6 to 10 carbon atoms; and R is selected from an unsaturated hydrocarbon group containing 2 to 4 carbon atoms, an aromatic group containing up to 14 carbon atoms, a heterocyclic group having one or two heteroatoms each selected from O, S or N and containing up to 14 carbon atoms or an N-oxide or acid addition salt of an N-heterocyclic group, a cyano group, a cycloalkenyl group containing 5 to 7 carbon atoms and a cycloalkyl group containing 3 to 10 carbon atoms, which groups are optionally substituted by one or more substituents selected from the groups hydroxy; cyano; halogen atoms having an atomic number of from 9 to 35 inclusive; alkyl, optionally substituted by hydroxy, amino, alkylcarbonylamino, alkoxy or alkylthio; haloalkyl, alkoxy, haloalkoxy, alkylthio, haloalkylthio, alkylsulfinyl, alkylsulfonyl, alkenyl or alkynyl of up to 4 carbon atoms; carboxyl; aminocarbonyl or amino, each of which can be substituted on the nitrogen by one or two alkyl groups of 1 to 4 carbon atoms; and stereoisomeric forms or mixtures thereof, which comprises reducing a lactone of formula IV as defined in claim 1 to provide a diol of formula V

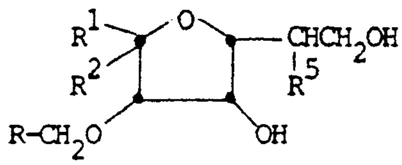

(V)

where R, $R^1$, $R^2$ and $R^5$ are as hereinbefore defined and subjecting the diol of formula V to conditions effecting ring closure to give a compound of formula I where $R^3$ and $R^4$ are each hydrogen.

**3.** A process according to claim 1 or 2 wherein the lactone of formula IV has been prepared by condensation of a glycol derivative of formula III

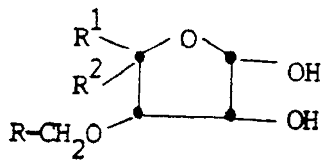

(III)

where R, $R^1$ and $R^2$ are as defined in claim 1, with a suitable condensing agent.

**4.** A process according to claim 3 wherein the glycol derivative of formula III has been obtained by acid hydrolysis of a corresponding furanose derivative.

**Revendications**

**Revendications pour les Etats contractants suivants : BE, CH, DE, FR, GB, GR, IT, LU, NL, SE**

**1.** Un composé de la formule I

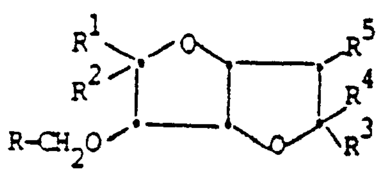

(I)

où $R^1$, $R^2$, $R^3$, $R^4$ et $R^5$, qui peuvent être indentiques ou différents, sont chacun indépendamment un atome d'hydrogène ou un groupe alcoyle contenant 1 à 5 atomes de carbone éventuellement substitué par un ou plusieurs atomes d'halogènes ayant un nombre atomique de 9 à 35 ; ou $R^3$ , $R^4$ et $R^5$ sont choisis en outre parmi un groupe alcényle ou alcynyle contenant 2 à 4 atomes de carbone et un groupe cycloaliphatique ou aryle mono- ou bicyclique contenant 6 à 10 atomes de carbone ; ou $R^3$ et $R^4$ en même temps que l'atome de carbone auquel ils sont attachés forment un noyau cycloaliphatique contenant 5 ou 6 atomes de carbone dans le noyau ; et R est choisi parmi un groupe d'hydrocarbure insaturé contenant 2 à 4 atomes de carbone, un groupe aromatique contenant jusqu'à 14 atomes de carbone, un groupe hétérocyclique ayant un ou deux hétéro-atomes choisis chacun parmi O, S ou N et contenant jusqu'à 14 atomes de carbone ou un N-oxyde ou un sel d'addition d'acide d'un groupe N-hétérocyclique, un groupe cyano, un groupe cycloalcényle contenant 5 à 7 atomes de carbone et un groupe cycloalcoyle contenant 3 à 10 atomes de carbone, ces groupes étant éventuellement substitués par un ou plusieurs substituants choisis parmi des groupes hydroxy ; des groupes cyano ; des atomes d'halogènes ayant un nombre atomique de 9 à 35 inclusivement ; des groupes alcoyle, éventuellement substitués par des groupes hydroxy, amino, alcoylcarbonylamino, alcoxy ou alcoylthio ; des groupes haloalcoyle, alcoxy, haloalcoxy, alcoylthio, haloalcoylthio, alcoylsulfinyle, alcoylsulfonyle, alcényle ou alcynyle ayant jusqu'à 4 atomes de carbone ; des groupes carboxyle ; des groupes aminocarbonyle ou amino dont chacun peut être substitué sur l'azote par un ou deux groupes alcoyle ayant de 1 à 4 atomes de carbone ; et des formes stéréoisomères ou leurs mélanges.

2. Un composé selon la revendication 1, dans lequel R est choisi parmi un groupe éthynyle ; un groupe phényle éventuellement substitué par un ou deux atomes d'halogènes d'un nombre atomique de 9 ou 17 ou par un groupe méthyle, et un groupe hétérocyclique pentagonal ou hexagonal contenant un ou deux hétéro-atomes choisis parmi O, S ou N, éventuellement substitué au noyau sur un atome de carbone par un halogène d'un nombre atomique de 9 ou 17 ou par un groupe alcoyle contenant 1 ou 2 atomes de carbone.

3. Un composé selon la revendication 2, dans lequel R est un groupe phényle, 2,6-difluorophényle, 2,6-dichlorophényle, 2-méthylphényle, 2-fluorophényle, tétrahydro-2-pyranyle, 6-chloro-2-pyridinyle ou 3-chloro-2-thiényle.

4. Un composé selon la revendication 1, 2 ou 3, dans lequel $R^1$, $R^2$, $R^3$, $R^4$ et $R^5$, qui peuvent être identiques ou différents, sont chacun indépendamment un atome d'hydrogène ou un groupe alcoyle contenant 1 ou 2 atomes de carbone éventuellement substitué par un ou plusieurs atomes d'halogènes.

5. Un composé selon l'une quelconque des revendications 1 à 4, dans lequel $R^1$ est un groupe méthyle ou éthyle.

6. Un composé selon l'une quelconque des revendications 1 à 5, dans lequel $R^2$, $R^3$, $R^4$ et $R^5$ sont chacun de l'hydrogène.

7. Un composé selon la revendication 1, dans lequel R est un groupe phényle, $R^1$ est un groupe éthyle et $R^2$, $R^3$, $R^4$ et $R^5$ sont chacun de l'hydrogène.

8. Un composé de la formule IV

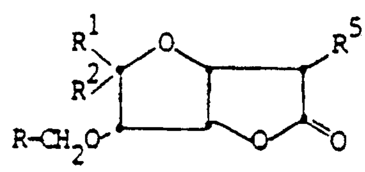

(IV)

où R, $R^1$, $R^2$ et $R^5$ sont tels que définis dans l'une quelconque des revendications 1 à 7 ; et des formes stéréoisomères ou leurs mélanges.

9. Un composé de la formule VI

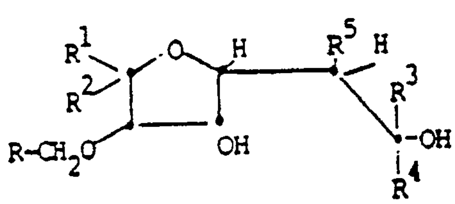

(VI)

où R, $R^1$, $R^2$, $R^3$, $R^4$ et R sont tels que définis dans l'une quelconque des revendications 1 à 7 et des formes stéréoisomères et leurs mélanges.

10. Un procédé de préparation d'un composé de formule I dans lequel $R^3$ et $R^4$ sont chacun de l'hydrogène, comprenant la réduction d'une lactone de formule IV telle que définie dans la revendication 8 pour donner un diol de formule V

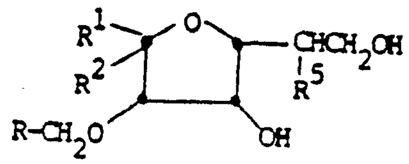

(V)

où R, $R^1$, $R^2$ et $R^5$ sont tels que définis dans la revendication 1, et l'exposition du diol de formule V à des conditions causant une cyclisation pour donner un composé de formule I dans lequel $R^3$ et $R^4$ sont chacun de l'hydrogène.

**11.** Un procédé de préparation d'un composé de formule I dans lequel $R^3$ et/ou $R^4$ sont autres que de l'hydrogène, comprenant la réaction d'une lactone de formule IV telle que définie dans la revendication 8 avec au moins un métal-alcoyle de formule $R^3M$, $R^4M$ ou $R^3R^4M$, où $R^3$ et $R^4$ sont tels que définis dans la revendication 1 autres que l'hydrogène et M représente un métal, éventuellement suivie d'un traitement par un agent réducteur, pour donner un composé de formule VI comme défini dans la revendication 9 dans lequel $R^3$ et/ou $R^4$ sont autres que de l'hydrogène, et l'exposition du diol de formule VI à des conditions effectuant une cyclisation pour donner un composé de formule I dans lequel $R^3$ et/ou $R^4$ sont autres que de l'hydrogène.

**12.** Un procédé selon la revendication 10 ou 11, dans lequel la lactone de formule IV a été préparée par condensation d'un dérivé glycol de formule III

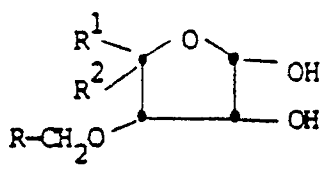

(III)

où R, $R^1$ et $R^2$ sont tels que définis dans la revendication 1, avec un agent de condensation approprié.

**13.** Un procédé selon la revendication 12, dans lequel le dérivé glycol de formule III a été obtenu par hydrolyse acide d'un dérivé de furanose correspondant.

**14.** Un composé de formule I quand il est préparé par le procédé de l'une quelconque des revendications 10 à 13.

**15.** Une composition pour le réglage de la croissance de plantes, qui comprend une quantité convenable pour réglage de la croissance de plantes d'un composé selon l'une quelconque des revendications 1 à 7 ou 14 et au moins un agent tensio-actif ou un véhicule.

**16.** Une composition pour le réglage de la croissance de plantes, qui comprend un composé selon l'une quelconque des revendications 1 à 7 ou 14 et au moins un agent tensio-actif et/ou un véhicule.

**17.** Une méthode pour le réglage de la croissance de plantes en un lieu, qui comprend l'application au lieu concerné d'un composé selon l'une quelconque des revendications 1 à 7 ou 14 ou d'une composition selon la revendication 15 ou 16.

**18.** Une méthode selon la revendication 17, où le réglage de la croissance a pour résultat la mort de la plante.

**Revendications pour l'Etat contractant suivant : AT**

**1.** Une composition pour le réglage de la croissance de plantes, comprenant au moins un agent tensio-actif ou véhicule et un composé de la formule I

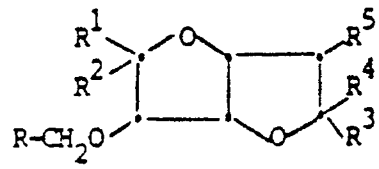

(I)

où $R^1$, $R^2$, $R^3$, $R^4$ et $R^5$, qui peuvent être identiques ou différents, sont chacun indépendamment un atome d'hydrogène ou un groupe alcoyle contenant 1 à 5 atomes de carbone éventuellement substitué par un ou plusieurs atomes d'halogènes ayant un nombre atomique de 9 à 35 ; ou $R^3$ , $R^4$ et $R^5$ sont choisis en outre parmi un groupe alcényle ou alcynyle contenant 2 à 4 atomes de carbone et un groupe cycloaliphatique ou aryle mono- ou bicyclique contenant 6 à 10 atomes de carbone ; ou $R^3$ et $R^4$ en même temps que l'atome de carbone auquel ils sont attachés forment un noyau cycloaliphatique contenant 5 ou 6 atomes de carbone dans le noyau ; et R est choisi parmi un groupe d'hydrocarbure insaturé contenant 2 à 4 atomes de carbone, un groupe aromatique contenant jusqu'à 14 atomes de carbone, un groupe hétérocyclique ayant un ou deux hétéro-atomes choisis chacun parmi O, S ou N et contenant jusqu'à 14 atomes de carbone ou un N-oxyde ou un sel d'addition d'acide d'un groupe N-hétérocyclique, un groupe cyano, un groupe cycloalcényle contenant 5 à 7 atomes de carbone et un groupe cycloalcoyle contenant 3 à 10 atomes de carbone, ces groupes étant éventuellement substitués par un ou plusieurs substituants choisis parmi des groupes hydroxy ; des groupes cyano ; des atomes d'halogènes ayant un nombre atomique de 9 à 35 inclusivement ; des groupes alcoyle éventuellement substitués par des groupes hydroxy, amino, alcoylcarbonylamino, alcoxy ou alcoylthio ; des groupes haloalcoyle, alcoxy, haloalcoxy, alcoylthio, haloalcoylthio, alcoylsulfinyle, alcoylsulfonyle, alcényle ou alcynyle ayant jusqu'à 4 atomes de carbone ; des groupes carboxyle ; des groupes aminocarbonyle ou amino dont chacun peut être substitué sur l'azote par un ou deux groupes alcoyle de 1 à 4 atomes de carbone ; ou des formes stéréoisomères ou leurs mélanges.

2. Une composition selon la revendication 1, dans laquelle, dans la formule I, R est choisi parmi un groupe éthynyle ; un groupe phényle éventuellement substitué par un ou deux atomes d'halogènes de nombre atomique 9 ou 17 ou par un groupe méthyle, et un groupe hétérocyclique pentagonal ou hexagonal contenant un ou deux hetéro-atomes choisis parmi O, S ou N, éventuellement substitué au noyau sur un atome de carbone par un halogène de nombre atomique 9 ou 17 ou par un groupe alcoyle contenant 1 ou 2 atomes de carbone.

3. Une composition selon la revendication 2, dans laquelle, dans la formule I, R est un groupe phényle, 2,6-difluorophényle, 2,6-dichlorophényle, 2-méthylphényle, 2-fluorophényle, tétrahydro-2-pyranyle, 6-chloro-2-pyridinyle ou 3-chloro-2-thiényle.

4. Une composition selon la revendication 1, 2 ou 3, dans laquelle, dans la formule I, $R^1$, $R^2$, $R^3$, $R^4$ et $R^5$, qui peuvent être identiques ou différents, sont chacun indépendamment un atome d'hydrogène ou un groupe alcoyle contenant 1 ou 2 atomes de carbone éventuellement substitué par un ou plusieurs atomes d'halogènes.

5. Une composition selon l'une quelconque des revendications 1 à 4, dans laquelle, dans la formule I, $R^1$ est un groupe méthyle ou éthyle.

6. Une composition selon l'une quelconque des revendications 1 à 5, dans laquelle, dans la formule I, $R^2$, $R^3$, $R^4$ et $R^5$ sont chacun de l'hydrogène.

7. Une composition selon la revendication 1, dans laquelle, dans la formule I, R est un groupe phényle, $R^1$ est un groupe éthyle et $R^2$, $R^3$, $R^4$ et $R^5$ sont chacun de l'hydrogène.

8. Une composition pour le réglage de la croissance de plantes, qui comprend une quantité convenable pour réglage de la croissance de plantes d'un composé de formule I tel que défini dans la revendication 1 et au moins un agent tensio-actif ou véhicule.

9. Un procédé pour la préparation d'un composé de formule I tel que défini dans la revendication 1 dans lequel $R^3$ et $R^4$ sont chacun de l'hydrogène, qui comprend la réduction d'une lactone de formule IV

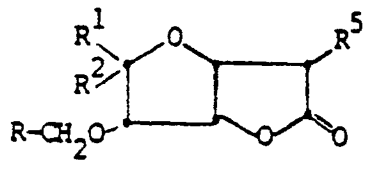

(IV)

pour donner un diol de formule V

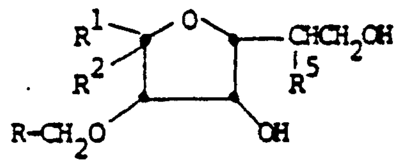

(V)

où R, $R^1$, $R^2$ et $R^5$ sont tels que définis dans la revendication 1 et l'exposition du diol de formule V à des conditions effectuant une cyclisation pour donner un composé de formule I dans lequel $R^3$ et $R^4$ sont chacun de l'hydrogène.

**10.** Un procédé pour la préparation d'un composé de formule I tel que défini dans la revendication 1 dans lequel $R^3$ et/ou $R^4$ sont autres que de l'hydrogène, qui comprend la réaction d'une lactone de formule IV telle que définie dans la revendication 9 avec au moins un métal-alcoyle de formule $R^3M$, $R^4M$ ou $R^3R^4M$, ou $R^3$ et $R^4$ sont tels que définis dans la revendication 1 autres que l'hydrogène et M représente un métal, éventuellement suivie d'un traitement par un agent réducteur, pour donner un composé de formule VI

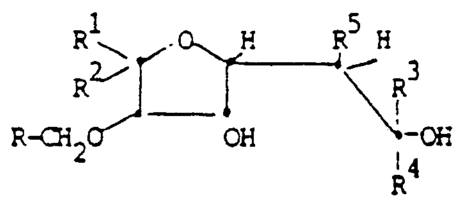

(VI)

où R, $R^1$, $R^2$ et $R^5$ sont tels que définis dans la revendication 1 et où $R^3$ et/ou $R^4$ sont autres que de l'hydrogène et l'exposition du diol de formule VI à des conditions effectuant une cyclisation pour donner un composé de formule I dans lequel $R^3$ et/ou $R^4$ sont autres que de l'hydrogène.

**11.** Un procédé selon la revendication 9 ou 10, dans lequel la lactone de formule IV a été préparée par condensation d'un dérivé glycol de formule III

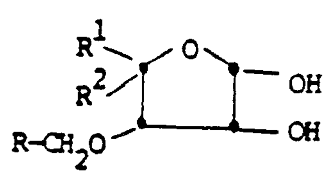

(III)

où R, $R^1$ et $R^2$ sont tels que définis dans la revendication 1 avec un agent de condensation approprié.

**12.** Un procédé selon la revendication 11, dans lequel le dérivé glycol de formule III a été obtenu par hydrolyse acide d'un dérivé de furanose correspondant.

**13.** Une méthode de réglage de la croissance de plantes en un lieu, qui comprend l'application, à ce lieu d'une composition selon l'une quelconque des revendications 1 à 8.

14. Une méthode selon la revendication 13, dans laquelle le réglage a pour résultat la mort de la plante.

**Revendications pour l'Etat contractant suivant : ES**

1. Un procédé de préparation d'un composé de la formule I

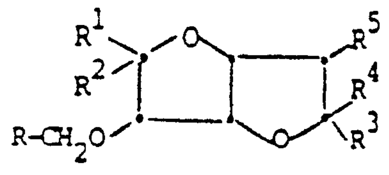

(I)

où $R^1$, $R^2$, $R^3$, $R^4$ et $R^5$, qui peuvent être identiques ou différents, sont chacun indépendamment un atome d'hydrogène ou un groupe alcoyle contenant 1 à 5 atomes de carbone éventuellement substitué par un ou plusieurs atomes d'halogènes ayant un nombre atomique de 9 à 35, avec la condition que $R^3$ et/ou $R^4$ sont autres que de l'hydrogène ; ou $R^3$, $R^4$ et $R^5$ sont choisis en outre parmi un groupe alcényle ou alcynyle contenant 2 à 4 atomes de carbone et un groupe cycloaliphatique ou aryle mono- ou bicyclique contenant 6 à 10 atomes de carbone ; ou $R^3$ et $R^4$ en même temps que l'atome de carbone auquel ils sont attachés forment un noyau cycloaliphatique contenant 5 ou 6 atomes de carbone dans le noyau ; et R est choisi parmi un groupe d'hydrocarbure insaturé contenant 2 à 4 atomes de carbone, un groupe aromatique contenant jusqu'à 14 atomes de carbone, un groupe hétérocyclique ayant un ou deux hétéro-atomes choisis chacun parmi O, S ou N et contenant jusqu'à 14 atomes de carbone ou un N-oxyde ou un sel d'addition d'acide d'un groupe N-hétérocyclique, un groupe cyano, un groupe cycloalcényle contenant 5 à 7 atomes de carbone et un groupe cycloalcoyle contenant 3 à 10 atomes de carbone, ces groupes étant éventuellement substitués par un ou plusieurs substituants choisis parmi des groupes hydroxy ; cyano ; des atomes d'halogènes ayant un nombre atomique de 9 à 35 inclusivement ; des groupes alcoyle, éventuellement substitués par des groupes hydroxy, amino, alcoylcarbonylamino, alcoxy ou alcoylthio ; des groupes haloalcoyle, alcoxy, haloalcoxy, alcoylthio, haloalcoylthio, alcoylsulfinyle, alcoylsulfonyle, alcényle ou alcynyle ayant jusqu'à 4 atomes de carbone ; des groupes carboxyle ; des groupes aminocarbonyle ou amino, qui peuvent être substitués chacun sur l'atome d'azote par un ou deux groupes alcoyle de 1 à 4 atomes de carbone ; et des formes stéréoisomères ou leurs mélanges, qui comprend la réaction d'une lactone de formule IV

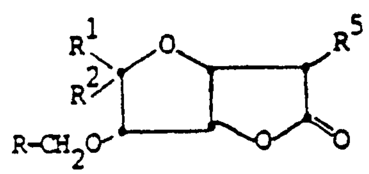

(IV)

avec au moins un métal-alcoyle de formule $R^3M$, $R^4M$ ou $R^3R^4M$, où R, $R^1$, $R^2$, $R^3$, $R^4$ et $R^5$ sont tels que définis ci-dessus et M représente un métal, éventuellement suivie d'un traitement par un agent réducteur, pour donner un composé de formule VI

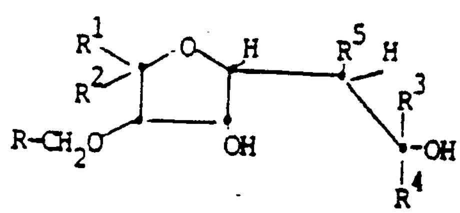

(VI)

et l'exposition du diol de formule VI à des conditions effectuant une cyclisation pour donner un composé de formule I.

2. Un procédé de préparation d'un composé de la formule I

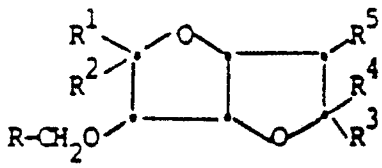

(I)

où $R^3$ et $R^4$ représentent chacun un atome d'hydrogène ; $R^1$, $R^2$, et $R^5$, qui peuvent être identiques ou différents, sont chacun indépendamment un atome d'hydrogène ou un groupe alcoyle contenant 1 à 5 atomes de carbone éventuellement substitué par un ou plusieurs atomes d'halogènes ayant un nombre atomique de 9 à 35 ; ou $R^5$ est choisi en outre parmi un groupe alcényle ou alcynyle contenant 2 à 4 atomes de carbone et un groupe cycloaliphatique ou aryle mono- ou bicyclique contenant 6 à 10 atomes de carbone ; et R est choisi parmi un groupe d'hydrocarbure insaturé contenant 2 à 4 atomes de carbone, un groupe aromatique contenant jusqu'à 14 atomes de carbone, un groupe hétérocyclique ayant un ou deux hétéro-atomes choisis chacun parmi O, S ou N et contenant jusqu'à 14 atomes de carbone ou un N-oxyde ou un sel d'addition d'acide d'un groupe N-hétérocyclique, un groupe cyano, un groupe cycloalcényle contenant 5 à 7 atomes de carbone et un groupe cycloalcoyle contenant 3 à 10 atomes de carbone, ces groupes étant éventuellement substitués par un ou plusieurs substituants choisis parmi des groupes hydroxy ; cyano ; des atomes d'halogènes ayant un nombre atomique de 9 à 35 inclusivement ; des groupes alcoyle éventuellement substitués par des groupes hydroxy, amino, alcoylcarbonylamino, alcoxy ou alcoylthio ; des groupes haloalcoyle, alcoxy, haloalcoxy, alcoylthio, haloalcoylthio, alcoylsulfinyle, alcoylsulfonyle, alcényle ou alcynyle ayant jusqu'à 4 atomes de carbone ; des groupes carboxyle ; des groupes aminocarbonyle ou amino, qui peuvent être substitués chacun sur l'azote par un ou deux groupes alcoyle de 1 à 4 atomes de carbone ; et des formes stéréoisomères ou leurs mélanges, qui comprend la réduction d'une lactone de formule IV telle que définie dans la revendication 1 pour donner un diol de formule V

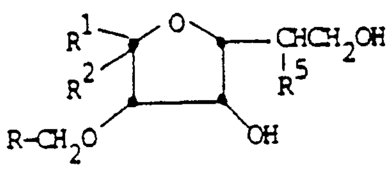

(V)

où R, $R^1$, $R^2$ et $R^5$ sont tels que définis ci-dessus et l'exposition du diol de formule V à des conditions effectuant une cyclisation pour donner un composé de formule I dans lequel $R^3$ et $R^4$ sont chacun de l'hydrogène.

3. Un procédé selon la revendication 1 ou 2, dans lequel la lactone de formule IV a été préparée par condensation d'un dérivé glycol de formule III

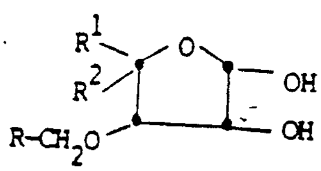

(III)

où R, $R^1$ et $R^2$ sont tels que définis dans la revendication 1 avec un agent de condensation approprié.

4. Un procédé selon la revendication 3, dans lequel le dérivé glycol de formule III a été obtenu par hydrolyse acide d'un dérivé de furanose correspondant.

**Patentansprüche**

**Patentansprüche für folgende Vertragsstaaten : BE, CH, DE, FR, GB, GR, IT, LI, LU, NL, SE**

1. Verbindung der Formel I

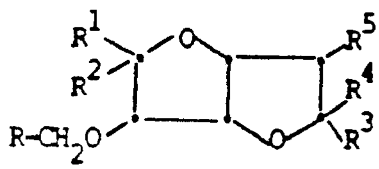

(I)

wobei $R^1$, $R^2$, $R^3$, $R^4$ und $R^5$, die gleich oder verschieden sein können, jeweils unabhängig voneinander ein Wasserstoffatom oder eine Alkylgruppe mit 1 bis 5 Kohlenstoffatomen, gegebenenfalls substituiert durch ein oder mehrere Halogenatome mit einer Ordnungszahl von 9 bis 35, bedeuten, oder $R^3$, $R^4$ und $R^5$ zusätzlich ausgewählt sind aus Alkenyl- oder Alkinylgruppen mit 2 bis 4 Kohlenstoffatomen und mono- oder bicyclischen cycloaliphatischen oder Arylgruppen mit 6 bis 10 Kohlenstoffatomen, oder $R^3$ und $R^4$ zusammen mit dem Kohlenstoffatom, an das sie gebunden sind, einen cycloaliphatischen Ring bilden, enthaltend 5 oder 6 Ring-Kohlenstoffatome, und R ausgewählt ist aus einer ungesättigten Kohlenwasserstoffgruppe mit 2 bis 4 Kohlenstoffatomen, einer aromatischen Gruppe mit bis zu 14 Kohlenstoffatomen, einer heterocyclischen Gruppe mit ein oder zwei Heteroatomen, ausgewählt aus O, S oder N und mit bis zu 14 Kohlenstoffatomen oder einem N-Oxid oder Säureadditionssalz einer N-heterocyclischen Gruppe, einer Cyanogruppe, einer Cycloalkenylgruppe, enthaltend 5 bis 7 Kohlenstoffatome, und einer Cycloalkylgruppe, enthaltend 3 bis 10 Kohlenstoffatome, wobei diese Gruppen gegebenenfalls substituiert sind durch einen oder mehrere Substituenten, ausgewählt aus den Gruppen Hydroxy, Cyano, Halogenatome mit einer Ordnungszahl von 9 bis 35 einschließlich, Alkyl, gegebenenfalls substituiert durch Hydroxy, Amino, Alkylcarbonylamino, Alkoxy oder Alkylthio, Halogenalkyl, Alkoxy, Halogenalkoxy, Alkylthio, Halogenalkylthio, Alkylsulfinyl, Alkylsulfonyl, Alkenyl oder Alkinyl mit bis zu 4 Kohlenstoffatomen, Carboxyl, Aminocarbonyl oder Amino, jeweils gegebenenfalls substituiert an dem Stickstoffatom durch eine oder zwei Alkylgruppen mit 1 bis 4 Kohlenstoffatomen, sowie stereoisomere Formen oder Gemische davon.

2. Verbindung nach Anspruch 1, wobei R ausgewählt ist aus einer Ethinylgruppe, einer Phenylgruppe, gegebenenfalls substituiert durch ein oder zwei Halogenatome mit einer Ordnungszahl von 9 oder 17 oder durch eine Methylgruppe, und einer 5- oder 6-gliedrigen hetererocyclischen Gruppe, enthaltend ein bis zwei heterocyclische Atome, ausgewählt aus O, S oder N, und gegebenenfalls am Ring an einem Kohlenstoffatom substituiert durch Halogen mit einer Ordnungszahl von 9 oder 17 oder durch Alkyl mit 1 bis 2 Kohlenstoffatomen.

3. Verbindung nach Anspruch 2, wobei R Phenyl, 2,6-Difluorphenyl, 2,6-Dichlorphenyl, 2-Methylphenyl, 2-Fluorphenyl, Tetrahydro-2-pyranyl, 6-Chlor-2-pyridinyl oder 3-Chlor-2-thienyl ist.

4. Verbindung nach Anspruch 1, 2 oder 3, wobei $R^1$, $R^2$, $R^3$, $R^4$ und $R^5$ , die gleich oder verschieden sein können, jeweils unabhängig voneinander ein Wasserstoffatom oder eine Alkylgruppe mit 1 oder 2 Kohlenstoffatomen, gegebenenfalls substituiert durch ein oder mehrere Halogenatome, bedeuten.

5. Verbindung nach einem der Ansprüche 1 bis 4, wobei $R^1$ Methyl oder Ethyl ist.

6. Verbindung nach einem der Ansprüche 1 bis 5, wobei $R^2$, $R^3$, $R^4$ und $R^5$ jeweils Wasserstoff sind.

7. Verbindung nach Anspruch 1, wobei R Phenyl, $R^1$ Ethyl und $R^2$, $R^3$, $R^4$ und $R^5$ jeweils Wasserstoff sind.

8. Verbindung der Formel IV

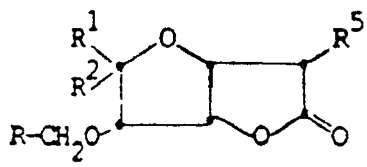

(IV)

wobei R, $R^1$, $R^2$ und $R^5$ wie in einem der Ansprüche 1 bis 7 definiert sind, sowie stereoisomere Formen oder Gemische davon.

**9.** Verbindung der Formel VI

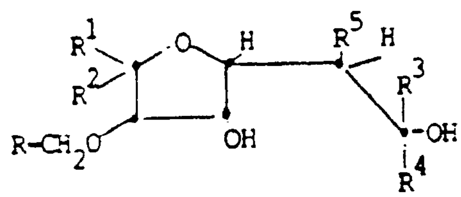

(VI)

wobei R, $R^1$, $R^2$, $R^3$, $R^4$ und $R^5$ wie in einem der Ansprüche 1 bis 7 definiert sind, sowie stereoisomere Formen und Gemische davon.

**10.** Verfahren zur Herstellung einer Verbindung der Formel I, wobei $R^3$ und $R^4$ jeweils Wasserstoff sind, umfassend die Reduktion eines Lactons der Formel IV, wie in Anspruch 8 definiert, unter Bildung eines Diols der Formel V

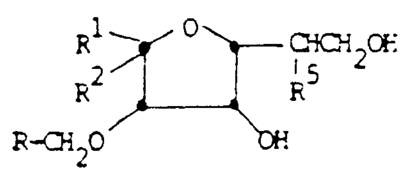

(V)

wobei R, $R^1$, $R^2$ und $R^5$ wie in Anspruch 1 definiert sind, und das Aussetzen des Diols der Formel V Bedingungen, die zu einem Ringschluß führen unter Bildung einer Verbindung der Formel I, in der $R^3$ und $R^4$ jeweils Wasserstoff sind.

**11.** Verfahren zur Herstellung einer Verbindung I, bei der $R^3$ und/oder $R^4$ eine andere Bedeutung als Wasserstoff haben, umfassend die Umsetzung eines Lactons der Formel IV, wie in Anspruch 8 definiert, mit mindestens einem Alkylmetall der Formel $R^3M$, $R^4M$ oder $R^3R^4M$, wobei $R^3$ und $R^4$ wie in Anspruch 1 definiert sind und nicht Wasserstoff bedeuten und M ein Metall bedeutet, und gegebenenfalls anschließende Behandlung mit einem Reduktionsmittel unter Bildung einer Verbindung der Formel VI, wie in Anspruch 9 definiert, wobei $R^3$ und/oder $R^4$ nicht Wasserstoff sind, und Aussetzen des Diols der Formel VI, Bedingungen, die zum Ringschluß führen unter Bildung einer Verbindung der Formel I, in der $R^3$ und/oder $R^4$ nicht Wasserstoff sind.

**12.** Verfahren nach Anspruch 10 oder 11, wobei das Lacton der Formel IV hergestellt worden ist durch Kondensation eines Glykol-Derivats der Formel III

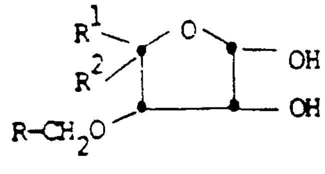

(III)

in der R, $R^1$ und $R^2$ wie in Anspruch 1 definiert sind, mit einem geeigneten Kondensationsmittel.

13. Verfahren nach Anspruch 12, wobei das Glykol-Derivat der Formel III erhalten worden ist durch saure Hydrolyse eines entsprechenden Furanose-Derivats.

14. Verbindung der Formel I, soweit sie hergestellt ist nach einem Verfahren nach einem der Ansprüche 10 bis 13.

15. Mittel zur Regulierung des Pflanzenwachstums, umfassend eine das Pflanzenwachstum regulierende Menge einer Verbindung nach einem der Ansprüche 1 bis 7 oder 14 und mindestens ein grenzflächenaktives Mittel oder einen Träger.

16. Mittel zur Regulierung des Pflanzenwachstums, umfassend eine Verbindung nach einem der Ansprüche 1 bis 7 oder 14 und mindestens ein grenzflächenaktives Mittel und/oder einen Träger.

17. Verfahren zur Regulierung des Pflanzenwachstums an einer Stelle, umfassend das Aufbringen einer Verbindung nach einem der Ansprüche 1 bis 7 oder 14 oder eines Mittels nach Anspruch 15 oder 16 auf die Stelle.

18. Verfahren nach Anspruch 17, wobei die Regulierung zu einer Abtötung der Pflanze führt.

**Patentansprüche für folgenden Vertragsstaat : AT**

1. Mittel zur Regulierung des Pflanzenwachstums, umfassend mindestens ein oberflächenaktives Mittel oder einen Träger und eine Verbindung der Formel I

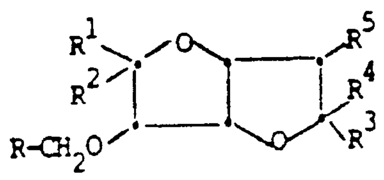

(I)

wobei $R^1$, $R^2$, $R^3$, $R^4$ und $R^5$, die gleich oder verschieden sein können, jeweils unabhängig voneinander ein Wasserstoffatom oder eine Alkylgruppe mit 1 bis 5 Kohlenstoffatomen, gegebenenfalls substituiert durch ein oder mehrere Halogenatome mit einer Ordnungszahl von 9 bis 35, bedeuten, oder $R^3$, $R^4$ und $R^5$ zusätzlich ausgewählt sind aus Alkenyl- oder Alkinylgruppen mit 2 bis 4 Kohlenstoffatomen und mono- oder bicyclischen cycloaliphatischen oder Arylgruppen mit 6 bis 10 Kohlenstoffatomen, oder $R^3$ und $R^4$ zusammen mit dem Kohlenstoffatom, an das sie gebunden sind, einen cycloaliphatischen Ring bilden, enthaltend 5 oder 6 Ring-Kohlenstoffatome, und R ausgewählt ist aus einer ungesättigten Kohlenwasserstoffgruppe mit 2 bis 4 Kohlenstoffatomen, einer aromatischen Gruppe mit bis zu 14 Kohlenstoffatomen, einer heterocyclischen Gruppe mit ein oder zwei Heteroatomen, ausgewählt aus O, S oder N und mit bis zu 14 Kohlenstoffatomen oder einem N-Oxid oder Säureadditionssalz einer N-heterocyclischen Gruppe, einer Cyanogruppe, einer Cycloalkenylgruppe, enthaltend 5 bis 7 Kohlenstoffatome, und einer Cycloalkylgruppe, enthaltend 3 bis 10 Kohlenstoffatome, wobei diese Gruppen gegebenenfalls substituiert sind durch einen oder mehrere Substituenten, ausgewählt aus den Gruppen Hydroxy, Cyano, Halogenatome mit einer Ordnungszahl von 9 bis 35 einschließlich, Alkyl, gegebenenfalls substituiert durch Hydroxy, Amino, Alkylcarbonylamino, Alkoxy oder Alkylthio, Halogenalkyl, Alkoxy, Halogenalkoxy, Alkylthio, Halogenalkylthio, Alkylsulfinyl, Alkylsulfonyl, Alkenyl oder Alkinyl mit bis zu 4 Kohlenstoffatomen, Carboxyl, Aminocarbonyl oder Amino, jeweils gegebenenfalls substituiert an dem Stickstoffatom durch eine oder zwei Alkylgruppen mit 1 bis 4 Kohlenstoffatomen, sowie stereoisomere Formen oder Gemische davon.

2. Mittel nach Anspruch 1, wobei bei der Formel I R ausgewählt ist aus einer Ethinylgruppe, einer Phenylgruppe, gegebenenfalls substituiert durch ein oder zwei Halogenatome mit einer Ordnungszahl von 9 oder 17 oder durch eine Methylgruppe, und einer 5- oder 6-gliedrigen heterercyclischen

Gruppe, enthaltend ein bis zwei heterocyclische Atome, ausgewählt aus O, S oder N, und gegebenenfalls am Ring an einem Kohlenstoffatom substituiert durch Halogen mit einer Ordnungszahl von 9 oder 17 oder durch Alkyl mit 1 bis 2 Kohlenstoffatomen.

3. Mittel nach Anspruch 2, wobei bei der Formel I R Phenyl, 2,6-Difluorphenyl, 2,6-Dichlorphenyl, 2-Methylphenyl, 2-Fluorphenyl, Tetrahydro-2-pyranyl, 6-Chlor-2-pyridinyl oder 3-Chlor-2-thienyl ist.

4. Mittel nach Anspruch 1, 2 oder 3, wobei bei der Formel I $R^1$, $R^2$, $R^3$, $R^4$ und $R^5$, die gleich oder verschieden sein können, jeweils unabhängig voneinander ein Wasserstoffatom oder eine Alkylgruppe mit 1 oder 2 Kohlenstoffatomen, gegebenenfalls substituiert durch ein oder mehrere Halogenatome, bedeuten.

5. Mittel nach einem der Ansprüche 1 bis 4, wobei in der Formel I $R^1$ Methyl oder Ethyl ist.

6. Mittel nach einem der Ansprüche 1 bis 5, wobei in der Formel $R^2$, $R^3$, $R^4$ und $R^5$ jeweils Wasserstoff sind.

7. Mittel nach Anspruch 1, wobei in der Formel I R Phenyl, $R^1$ Ethyl und $R^2$, $R^3$, $R^4$ und $R^5$ jeweils Wasserstoff sind.

8. Mittel zur Regulierung des Pflanzenwachstums, umfassend eine das Pflanzenwachstum regulierende Menge einer Verbindung der Formel I, wie in Anspruch 1 definiert, und mindestens ein oberflächenaktives Mittel oder einen Träger.

9. Verfahren zur Herstellung einer Verbindung der Formel I, wie in Anspruch 1 definiert, wobei $R^3$ und $R^4$ jeweils Wasserstoff sind, umfassend die Reduktion eines Lactons der Formel IV

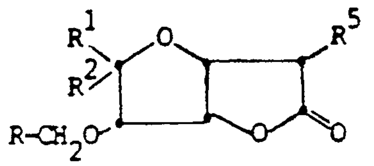

(IV)

unter Bildung eines Diols der Formel V

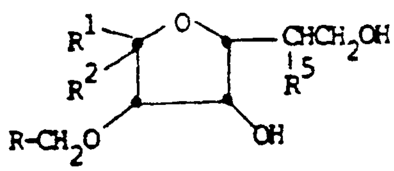

(V)

wobei R, $R^1$, $R^2$ und $R^5$ wie in Anspruch 1 definiert sind, und das Aussetzen des Diols der Formel V, Bedingungen, die zu einem Ringschluß führen unter Bildung einer Verbindung der Formel I, in der $R^3$ und $R^4$ jeweils Wasserstoff sind.

10. Verfahren zur Herstellung einer Verbindung I, wie in Anspruch 1 definiert, bei der $R^3$ und/oder $R^4$ eine andere Bedeutung als Wasserstoff haben, umfassend die Umsetzung eines Lactons der Formel IV, wie in Anspruch 9 definiert, mit mindestens einem Alkylmetall der Formel $R^3$ M, $R^4$ M oder $R^3$ $R^4$ M, wobei $R^3$ und $R^4$ wie in Anspruch 1 definiert sind und nicht Wasserstoff bedeuten und M ein Metall bedeutet, und gegebenenfalls anschließende Behandlung mit einem Reduktionsmittel unter Bildung einer Verbindung der Formel VI

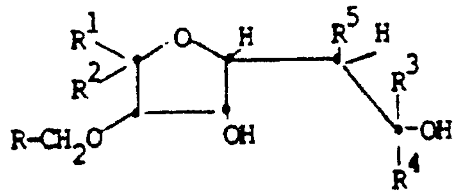

(VI)

wobei R, $R^1$, $R^2$ und $R^5$ wie in Anspruch 1 definiert sind und wobei $R^3$ und/oder $R^4$ nicht Wasserstoff sind, und Aussetzen des Diols der Formel VI Bedingungen, die zum Ringschluß führen unter Bildung einer Verbindung der Formel I, in der $R^3$ und/oder $R^4$ nicht Wasserstoff sind.

**11.** Verfahren nach Anspruch 9 oder 10, wobei das Lacton der Formel IV hergestellt worden ist durch Kondensation eines Glykol-Derivats der Formel III

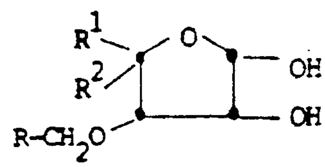

(III)

in der R, $R^1$ und $R^2$ wie in Anspruch 1 definiert sind, mit einem geeigneten Kondensationsmittel.

**12.** Verfahren nach Anspruch 11, wobei das Glykol-Derivat der Formel III erhalten worden ist durch saure Hydrolyse eines entsprechenden Furanose-Derivats.

**13.** Verfahren zur Regulierung des Pflanzenwachstums an einer Stelle, umfassend das Aufbringen eines Mittels nach einem der Ansprüche 1 bis 8 auf die Stelle.

**14.** Verfahren nach Anspruch 13, wobei die Regulierung zu einer Abtötung der Pflanze führt.

**Patentansprüche für folgenden Vertragsstaat : ES**

**1.** Verfahren zur Herstellung einer Verbindung der Formel I

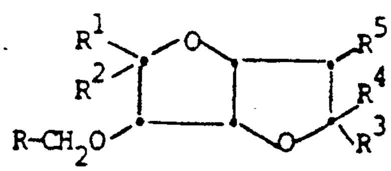

(I)

wobei $R^1$, $R^2$, $R^3$, $R^4$ und $R^5$, die gleich oder verschieden sein können, jeweils unabhängig voneinander ein Wasserstoffatom oder eine Alkylgruppe mit 1 bis 5 Kchlenstoffatomen, gegebenenfalls substituiert durch ein oder mehrere Halogenatome mit einer Ordnungszahl von 9 bis 35, bedeuten, mit der Maßgabe, daß $R^3$ und/oder $R^4$ kein Wasserstoff ist, oder $R^3$, $R^4$ und $R^5$ zusätzlich ausgewählt sind aus Alkenyl- oder Alkinylgruppen mit 2 bis 4 Kohlenstoffatomen und mono- oder bicyclischen cycloaliphatischen oder Arylgruppen mit 6 bis 10 Kohlenstoffatomen, oder $R^3$ und $R^4$ zusammen mit dem Kohlenstoffatom, an das sie gebunden sind, einen cycloaliphatischen Ring bilden, enthaltend 5 oder 6 Ring-Kohlenstoffatome, und R ausgewählt ist aus einer ungesättigten Kohlenwasserstoffgruppe mit 2 bis 4 Kohlenstoffatomen, einer aromatischen Gruppe mit bis zu 14 Kohlenstoffatomen, einer heterocyclischen Gruppe mit ein oder zwei Heteroatomen, ausgewählt aus O, S oder N und mit bis zu 14 Kohlenstoffatomen oder einem N-Oxid oder Säureadditionssalz einer N-heterocyclischen Gruppe, einer Cyanogruppe, einer Cycloalkenylgruppe, enthaltend 5 bis 7 Kohlenstoffatome, und einer Cycloalkylgruppe, enthaltend 3 bis 10 Kohlenstoffatome, wobei diese Gruppen gegebenenfalls substituiert sind durch einen oder mehrere Substituenten, ausgewählt aus den Gruppen Hydroxy, Cyano, Halogenatome mit einer Ordnungszahl von 9 bis 35 einschließlich, Alkyl, gegebenenfalls substituiert durch Hydroxy,

Amino, Alkylcarbonylamino, Alkoxy oder Alkylthio, Halogenalkyl, Alkoxy, Halogenalkoxy, Alkylthio, Halogenalkylthio, Alkylsulfinyl, Alkylsulfonyl, Alkenyl oder Alkinyl mit bis zu 4 Kohlenstoffatomen, Carboxyl, Aminocarbonyl oder Amino, jeweils gegebenenfalls substituiert an dem Stickstoffatom durch eine oder zwei Alkylgruppen mit 1 bis 4 Kohlenstoffatomen, sowie stereoisomere Formen oder Gemische davon, umfassend die Umsetzung eines Lactons der Formel IV

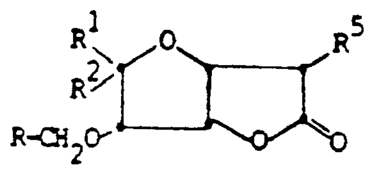

(IV)

mit mindestens einem Alkylmetall der Formel $R^3$ M, $R^4$M oder $R^3R^4$M, wobei R, $R^1$, $R^2$, $R^3$, $R^4$ und $R^5$ wie oben definiert sind und M ein Metall bedeutet, und gegebenenfalls anschließende Behandlung mit einem Reduktionsmittel unter Bildung einer Verbindung der Formel VI

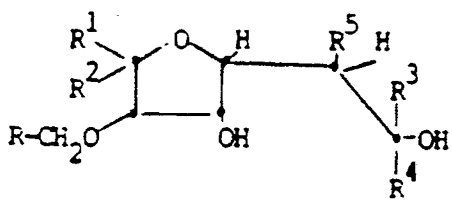

(VI)

und Aussetzen des Diols der Formel VI, Bedingungen, die zum Ringschluß führen unter Bildung einer Verbindung der Formel I.

2. Verfahren zur Herstellung einer Verbindung der Formel I

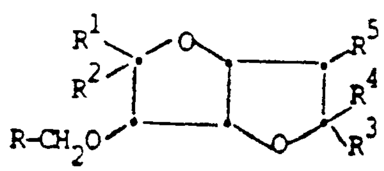

(I)

in der $R^3$ und $R^4$ jeweils ein Wasserstoffatom bedeuten, $R^1$, $R^2$ und $R^5$, die gleich oder verschieden sein können, jeweils unabhängig voneinander ein Wasserstoffatom oder eine Alkylgruppe mit 1 bis 5 Kohlenstoffatomen, gegebenenfalls substituiert durch ein oder mehrere Halogenatome mit einer Ordnungszahl von 9 bis 35, bedeuten, oder $R^5$ zusätzlich ausgewählt ist aus Alkenyl- oder Alkinylgruppen mit 2 bis 4 Kohlenstoffatomen und mono- oder bicyclischen cycloaliphatischen oder Arylgruppen mit 6 bis 10 Kohlenstoffatomen und R ausgewählt ist aus einer ungesättigten Kohlenwasserstoffgruppe mit 2 bis 4 Kohlenstoffatomen, einer aromatischen Gruppe mit bis zu 14 Kohlenstoffatomen, einer heterocyclischen Gruppe mit ein oder zwei Heteroatomen, ausgewählt aus O, S oder N und mit bis zu 14 Kohlenstoffatomen oder einem N-Oxid oder Säureadditionssalz einer N-heterocyclischen Gruppe, einer Cyanogruppe, einer Cycloalkenylgruppe, enthaltend 5 bis 7 Kohlenstoffatome, und einer Cycloalkylgruppe, enthaltend 3 bis 10 Kohlenstoffatome, wobei diese Gruppen gegebenenfalls substituiert sind durch einen oder mehrere Substituenten, ausgewählt aus den Gruppen Hydroxy, Cyano, Halogenatome mit einer Ordnungszahl von 9 bis 35 einschließlich, Alkyl, gegebenenfalls substituiert durch Hydroxy, Amino, Alkylcarbonylamino, Alkoxy oder Alkylthio, Halogenalkyl, Alkoxy, Halogenalkoxy, Alkylthio, Halogenalkylthio, Alkylsulfinyl, Alkylsulfonyl, Alkenyl oder Alkinyl mit bis zu 4 Kohlenstoffatomen, Carboxyl, Aminocarbonyl oder Amino, jeweils gegebenenfalls substituiert an dem Stickstoffatom durch eine oder zwei Alkylgruppen mit 1 bis 4 Kohlenstoffatomen, sowie stereoisomere Formen oder Gemische davon, umfassend die Reduktion eines Lactons der Formel IV, wie in Anspruch 1 definiert, unter Bildung eines Diols der Formel V

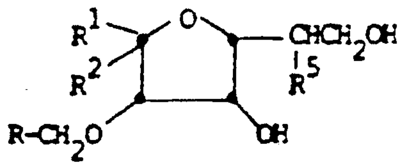

(V)

wobei R, $R^1$, $R^2$ und $R^5$ wie oben definiert sind, und das Aussetzen des Diols der Formel V,Bedingungen, die zu einem Ringschluß führen unter Bildung einer Verbindung der Formel I, in der $R^3$ und $R^4$ jeweils Wasserstoff sind.

3. Verfahren nach Anspruch 1 oder 2, wobei das Lacton der Formel IV hergestellt worden ist durch Kondensation eines Glykol-Derivats der Formel III

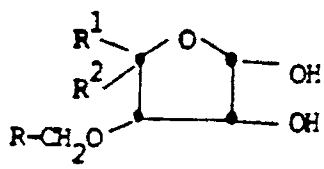

(III)

in der R, $R^1$ und $R^2$ wie in Anspruch 1 definiert sind, mit einem geeigneten Kondensationsmittel.

4. Verfahren nach Anspruch 12, wobei das Glykol-Derivat der Formel III erhalten worden ist durch saure Hydrolyse eines entsprechenden Furanose-Derivats.